# FASCICULE DE BREVET EUROPEEN

(11) **EP 0 860 160 B1**
(45) Date de publication et mention de la délivrance du brevet: **26.04.2000**
(21) Numéro de dépôt: 98490004.3
(22) Date de dépôt: 14.01.1998
(51) Int. Cl.: A61F 13/62

(54) **Article d'hygiène comportant un système d'attache mécanique**
Hygienischer Artikel mit einem Flächenreissverschluss
Hygiene article with a mechancial fastening system

(30) Priorité: 14.01.1997 FR 9700504
(43) Date de publication de la demande: 26.08.1998
(73) Titulaire: Tape Industries, 59160 Capinghem (FR)
(72) Inventeur: Moreau, Patrick, 59310 Beuvry La Foret (FR)
(74) Mandataire: Hénnion, Jean-Claude

(56) Documents cités:
- EP-A- 0 235 014
- EP-A- 0 517 204
- EP-A- 0 704 197
- GB-A- 2 284 742

## Description

La présente invention concerne les systèmes d'attaches dénommés de manière classique attaches mécaniques , comportant une bande présentant en surface des éléments mâles , ayant notamment la forme de crochets, de harpons ou de têtes de clous et une autre bande présentant en surface des éléments femelles, ayant notamment la forme de boucles. Lorsque l'on applique les deux bandes l'une contre l'autre, les éléments mâles pénètrent dans les éléments femelles de sorte que les deux bandes sont difficilement séparables lorsque l'on exerce des forces de traction sensiblement dans le plan des deux bandes, par contre les deux bandes sont séparables lorsque l'on exerce une force de traction essentiellement perpendiculaire ou en oblique par rapport aux deux bandes. La présente invention concerne plus particulièrement un article d'hygiène équipé d'un système d'attache mécanique dont les pattes d'attache comportent une bande revêtue d'éléments femelles.

Le principe du système d'attaches mécaniques est maintenant bien connu et largement utilisé, s'agissant notamment des produits diffusés sous la marque déposée Velcro®. Ces systèmes d'attaches sont mis en oeuvre dans des domaines aussi divers que l'habillement , la chaussure, l'ameublement... Les deux bandes constitutives du système d'attaches sont fixées sur des pièces que l'on veut solidariser l'une à l'autre de manière temporaire, avec possibilité d'ouvertures et de fermetures multiples.

Dans la plupart de ces applications , il est souhaitable que le système d'attaches mécaniques ait de bonnes propriétés de résistance et de durabilité , fonction de la durée de vie normale de l'article auquel il est attaché.

Cependant ce type de système d'attaches mécaniques est maintenant mis en oeuvre dans des articles jetables tels que des articles d'hygiène , en particulier des couches-culottes , dans lesquelles le système d'attaches utilisé jusqu'à présent était un système d'attaches adhésives. De manière conventionnelle, dans un article d'hygiène du type couche-culotte, comprenant une feuille extérieure imperméable, une feuille intérieure perméable et un matelas absorbant disposé entre lesdites feuilles extérieure et intérieure, la fermeture de l'article autour de la taille de l'usager était réalisée par deux systèmes d'attaches aptes à réaliser la fermeture des deux côtés de l'article. Dans un mode connu de réalisation, chaque système comprend une patte d'attache , fixée latéralement selon un bord de la partie arrière de la feuille extérieure et également un élément frontal sur la partie avant de la feuille extérieure, permettant de rigidifier cette partie avant et permettant le collage et le décollage répétés de la patte d'attache adhésive. Selon l'enseignement du document FR-A-2.534.781, l'élément frontal est une bande unique en matière plastique collée sensiblement sur toute la largeur de la partie avant de la feuille extérieure.

Par analogie avec l'expérience des systèmes d'attaches adhésives, les producteurs de couches-culottes ont disposé sur les pattes d'attaches latérales les éléments mâles du système d'attaches mécaniques. Quant aux éléments femelles dudit système, ils sont rapportés , par exemple sous la forme d'une bande unique transversale sur la partie avant de la feuille extérieure. Cette disposition permet , avec une faible quantité d'éléments mâles sur la patte d'attache latérale d' obtenir un serrage adapté autour de la taille de l'usager.

Selon l'enseignement du document EP-A-0 235 014, il est proposé de disposer sur les pattes d'attaches latérales les éléments femelles et sur la partie avant de la feuille extérieure les éléments mâles. On peut en effet s'attendre dans cette configuration à un certain nombre de désagréments. La présence des éléments mâles donne à la bande qui la supporte une rigidité très importante, ce qui pourrait constituer une gêne pour l'usager, si la bande était appliquée comme précédemment sur quasiment toute la largeur de la partie avant de la feuille imperméable. Si pour limiter ou éviter ce désagrément, on cherche à réduire la largeur de la bande supportant les éléments mâles , on a alors une perte de résistance mécanique pouvant entraîner une déchirure lors de l'ouverture du système d'attaches.

L'objet de l'invention vise à remédier à ces inconvénients. Il concerne un article d'hygiène du type couche-culotte, selon la revendication indépendante 1.

Dans une première variante de réalisation, les moyens mâles d'attaches sont rapportés sur la bande de renforcement, celle-ci étant fixée sur la face externe de la feuille extérieure.

Dans une seconde variante de réalisation, l'élément frontal est fixé sur la face externe de la feuille extérieure et la bande de renforcement est fixée sur la face interne de la feuille extérieure.

La présente invention sera mieux comprise à la lecture de la description qui va être faite d'un exemple de réalisation d'une patte d'attache, porteuse d'éléments femelles du type boucles , pour système d'attaches mécaniques et d'une couche-culotte équipée de tels systèmes d'attaches, illustrée par le dessin annexé dans lequel:
- La figure 1 est une représentation schématique en coupe des trois bandes à superposer pour constituer la patte d'attache,
- La figure 2 est une représentation schématique en coupe de la patte d'attache composée des trois bandes de la figure 1,
- La figure 3 est une représentation schématique en coupe d'une patte d'attache d'un autre mode de réalisation,
- La figure 4 est une représentation schématique en coupe de la patte d'attache de la figure 2 mise en place selon le bord latéral de la partie arrière de la couche-culotte,
- La figure 5 est une représentation schématique en coupe de la patte d'attache de la figure 4 coopérant avec les éléments mâles placés sur la partie avant de la couche et
- La figure 6 est une représentation en perspective d'une couche-culotte équipée de systèmes d'attaches mécaniques selon l'invention.

On connaît l'utilisation de systèmes d'attaches mécaniques pour la fermeture d'articles d'hygiène du type couches-culottes. Dans tous les modes de réalisation connus un tel système comprend une patte d' attache qui est fixé latéralement selon un bord de la partie arrière de la feuille extérieure et qui comporte des éléments mâles d'attaches mécaniques, du type crochets , harpons , têtes de clous. Ces éléments mâles coopèrent, pour la fermeture du côté de la couche-culotte avec les éléments femelles qui sont portés sur la partie avant de la feuille extérieure. Ces éléments femelles du type boucles, sont constitués par exemple par un non-tissé ou par un textile tricoté ou gratté qui est collé sous forme d'une bande transversale sur toute la largeur de la partie avant de la feuille extérieure de la couche-culotte.

La caractéristique originale de la présente invention consiste dans le fait que la patte d'attache 1 , qui est fixée selon un bord latéral 2 de la partie arrière 3 de la feuille extérieure 4 comporte des éléments femelles 5 d'attaches mécaniques , c'est-à-dire des éléments en forme de boucles. Par contre la partie avant 6 de la feuille extérieure 4 est pourvue d'éléments mâles 7.

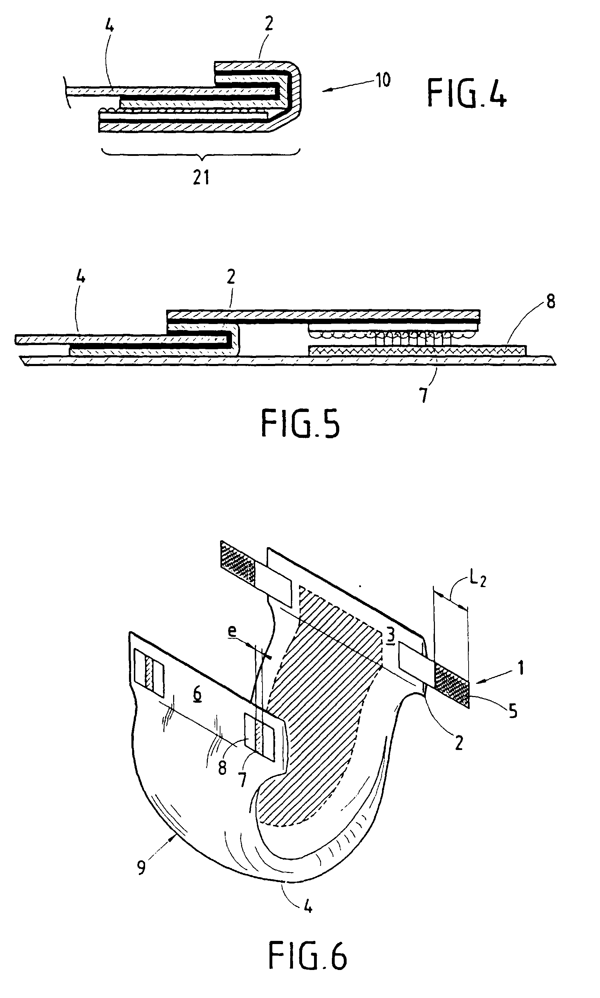

Dans l'exemple illustré à la figure 6 ces éléments mâles 7 sont constitués d'une bande de faible largeur e qui est disposée sensiblement dans la partie centrale d'une bande de renforcement 8 qui est une feuille en matière plastique, elle-même collée sur la face externe de la feuille imperméable 4 dans la partie avant 6.

La présence de la bande de renforcement 8 permet d'éviter les risques de déchirure de la feuille extérieure 4 lors de la réouverture du système d'attaches mécaniques.

Bien sûr il serait possible de mettre en oeuvre une bande porteuse d'éléments mâles 7 de plus grande largeur mais ceci ne présente pas d'avantages particuliers, mais au contraire présente d'une part l'inconvénient d'un coût plus élevé et d'autre part l'inconvénient d'augmenter localement la rigidité de la partie avant 6 de la couche. S'agissant du coût de production, il est à souligner que le système d'attache selon l'invention s'avère moins cher globalement qu'un système d'attache avec les éléments mâles portés sur la patte d'attache, du fait d'une moindre quantité d'éléments femelles.

Il est à noter que la largeur L₂ de la bande porteuse d'éléments femelles 5 doit être nettement supérieure à la largeur e de la bande porteuse d'éléments mâles , si l'on veut pouvoir réaliser un ajustement précis de la couche-culotte 9 autour de la taille de l'usager. La faculté de réglage s'apprécie donc en fonction de la différence de largeur (L₂ - e).

La bande de renforcement 8 qui , dans l'exemple illustré à la figure 6, est collée sur la face externe de la feuille imperméable peut éventuellement être collée sur la face interne de la même feuille imperméable 4, tandis que la bande porteuse des éléments mâles 7 est bien sûr obligatoirement sur la face externe.

On a représenté sur la figure 6 une seule bande porteuse d'éléments mâles 7 disposée verticalement et de faible largeur e, mais toutes les variantes de réalisation sont possibles : plusieurs bandes de petite largeur espacées les unes par rapport aux autres, une ou plusieurs bandes disposées horizontalement ...

La présente invention concerne également une patte d'attache pour article d'hygiène du type couche-culotte, patte d'attache qui est destinée à être fixée latéralement selon un bord de la partie arrière de l'article et comportant des éléments femelles d'attaches mécaniques , du type boucles.

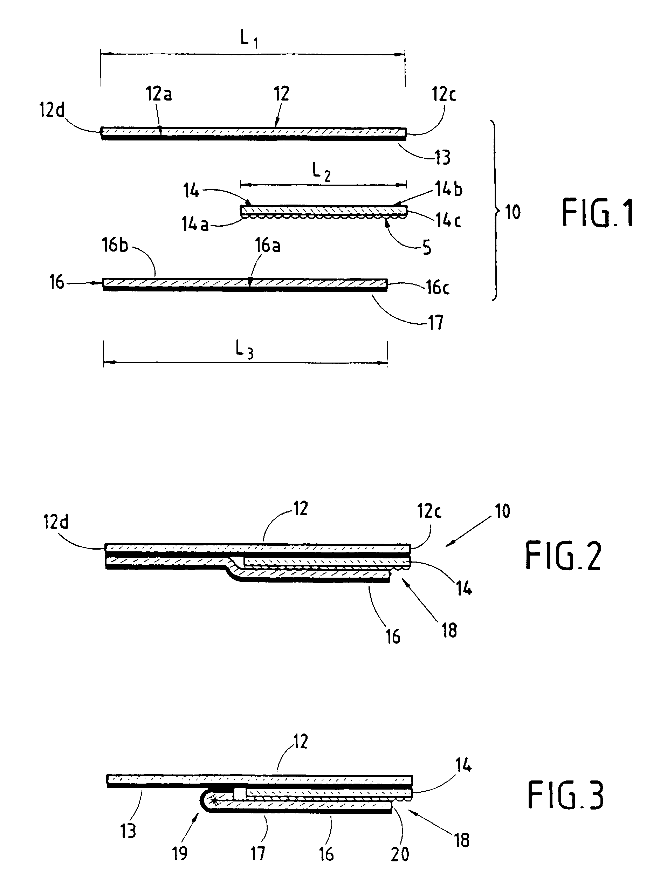

Cette patte d'attache 10 est composée par la superposition de trois bandes 12,14 et 16. On a représenté sur la figure 1 les trois dites bandes séparées les unes des autres. En pratique, les trois bandes préconstituées sont alimentées en continu, superposées et tronçonnées pour constituer la patte d'attache 10.

La première bande 12 a une largeur L₁ et présente sur sa première face 12a un premier revêtement adhésif 13.

La deuxième bande 14 a une largeur L₂ qui est inférieure à L₁ et présente sur sa première face 14a des boucles 5 constituant les éléments femelles du système d'attaches mécaniques.

La troisième bande 16 a une largeur L₃ qui est inférieure à L₁ et présente sur sa première face 16a un second revêtement adhésif 17.

Comme illustré à la figure 1, la deuxième bande 14 est prise en sandwich entre la première bande 12 et la troisième 16. La seconde face 14b de la deuxième bande 14 est en contact avec le premier revêtement adhésif 13 de la première bande 12, tandis que les boucles 5 sont en contact avec la seconde face 16b, exempte de revêtement adhésif , de la troisième bande 16.

Lorsque ces trois bandes sont superposées , on obtient la patte d'attache 10 telle qu'illustrée à la figure 2.

La deuxième bande 14 est appliquée sur la première bande 12 depuis le premier bord 12c de celle-ci. Du fait que la largeur L₂ de la deuxième bande 14 est inférieure à celle L₁ de la première bande 12, la troisième bande 16 est appliquée sur la première bande 12 sur la portion de ladite première bande 12 non recouverte par la deuxième bande 14. Plus précisément on applique la troisième bande 16 sur la première bande 12 depuis le second bord 12d de la première bande 12. Du fait que la longueur L₃ de la troisième bande 16 est inférieure à celle L₁ de la première bande 12, il subsiste un espace 18 dans lequel les deux premières bandes 12,14 ne sont pas recouvertes par la troisième bande 16. Cet espace fait office de languette de préhension lors de la mise en oeuvre de la patte d'attache sur la couche-culotte 9.

A la figure 3 on a illustré une autre variante de réalisation qui diffère de la première variante par la disposition particulière de la troisième bande 16. Celle-ci est d'une longueur inférieure à celle du premier exemple et a son bord 19, opposé à celui 20 proche de l'espace 18 qui est replié de sorte que c'est le second revêtement adhésif 17 qui est appliqué sur le premier revêtement adhésif 13 de la première bande 12. Cette configuration particulière permet d'utiliser une quantité moindre de troisième bande 16.

La mise en place de la patte d'attache 10 sur le bord latéral de la partie arrière de la feuille extérieure 4 est réalisée automatiquement sur les machines de production. Lors de cette mise en place , la patte d'attache 10 est fixée sur la feuille extérieure 4 de part et d'autre du bord latéral 2, ladite feuille étant repliée sur elle-même de manière à s'appliquer sur chacune des faces externe et interne de ladite feuille 4.

Le collage de la patte d'attache 10 sur la feuille 4 est obtenu grâce au second revêtement adhésif 17 de la troisième bande 16 et éventuellement, dans le cas de la variante de la figure 3, grâce au premier revêtement adhésif 13 de la première bande 12. Sur la figure 4 on a illustré le positionnement de la patte d'attache 10 selon la variante de la figure 2.

Le maintien en position, à l'état replié , de la partie 21 de la patte d'attache 10 qui se trouve sur la face interne de la feuille 4 est obtenu notamment par la présence de zones évidées dans la deuxième bande 14 grâce auxquelles l'on peut avoir une solidarisation, minime mais suffisante, entre la première 12 et la troisième 16 bandes grâce au premier revêtement adhésif 13 de la première bande 12.

Lors de l'utilisation de la patte d'attache 10, l'opérateur se saisit des deux bandes 12 , 14 au niveau de l'espace 18, comme d'une languette de préhension, et ouvre ainsi la patte 10. Il applique ensuite ladite patte 10 au droit de la bande 8 sur laquelle sont disposés les éléments mâles 7. Il y a interpénétration des éléments mâles 7 dans les boucles 5, comme cela est bien connu dans tout système d'attaches mécaniques. On obtient donc la fermeture du côté de la couche-culotte 9 et sa réouverture possible grâce au système d'attaches de l'invention comprenant la patte d'attache 10 et les éléments mâles 7 disposés sur la partie avant 6 de la couche-culotte 9.

## Revendications

1. Article d'hygiène (9) du type couche-culotte , comprenant une feuille extérieure (4) imperméable, une feuille intérieure perméable, un matelas absorbant disposé entre les deux dites feuilles , et deux systèmes d'attaches permettant de refermer l'article autour de la taille de l'usager, chaque système comprenant une patte d'attache fixée latéralement selon un bord de la partie arrière de la feuille extérieure et au moins un élément frontal sur la partie avant de la feuille extérieure , apte à coopérer avec ladite patte d'attache, s'agissant d'un système d'attaches mécaniques, la patte d'attache (1) est pourvue d'éléments femelles (5), notamment du type boucles , et l'élément frontal est pourvu d'éléments mâles (7), caractérisé en ce que l'élément frontal est de faible largeur (e), laquelle est nettement inférieure à la largeur L₂ de la bande porteuse d'éléments femelles constituant la patte d'attache (1); et en ce que le système d'attache comprend au moins une bande de renforcement (8) qui est fixée sur la partie avant (6) de la feuille extérieure (4) , au droit de (ou des) l'élément frontal (aux) et sur une largeur supérieure à ce (ou ces) dernier(s).

2. Article d'hygiène selon la revendication 1, caractérisé en ce que les moyens mâles d'attaches (7) sont rapportés sur la bande de renforcement (8), celle-ci étant fixée sur la face externe de la feuille extérieure (4).

3. Article d'hygiène selon la revendication 1, caractérisé en ce que l'élément frontal est fixé sur la face externe de la feuille extérieure et la bande de renforcement est fixée sur la face interne de la feuille extérieure.

## Patentansprüche

1. Hygieneartikel (9) der Art Windelhöschen, der eine undurchlässige äußere Folie (4), eine durchlässige innere Folie, ein zwischen den beiden Folien angeordnetes absorbierendes Kissen und zwei Befestigungssysteme aufweist, die es ermöglichen, den Artikel um die Taille des Benutzers herum zu schließen, wobei jedes System eine Befestigungslasche, die seitlich entlang eines Rands des rückwärtigen Bereichs der äußeren Folie befestigt wird, und mindestens ein stirnseitiges Element auf dem vorderen Bereich der äußeren Folie aufweist, das mit der Befestigungslasche zusammenwirken kann, wobei es sich um ein mechanisches Befestigungssystem handelt, bei dem die Befestigungslasche (1) mit Aufnahmeelementen (5) insbesondere der Art Schlaufen und das stirnseitige Element mit Eingreifelementen (7) versehen ist, dadurch gekennzeichnet, daß das stirnseitige Element eine geringe Breite (e) aufweist, die deutlich kleiner ist als die Breite L₂ des die Befestigungslasche (1) bildenden und die Aufnahmeelemente tragenden Bands; und daß das Befestigungssystem mindestens ein Verstärkungsband (8) aufweist, das auf dem vorderen Bereich (6) der äußeren Folie (4) vor dem (oder den) stirnseitigen Element(en) (6) über eine Breite befestigt ist, die größer ist als dieses Element (oder diese Elemente).

2. Hygieneartikel nach Anspruch 1, dadurch gekennzeichnet, daß die Eingreifmittel (7) der Befestigungen auf das Verstärkungsband (8) aufgebracht sind, das selbst auf der Außenseite der äußeren Folie (4) befestigt ist.

3. Hygieneartikel nach Anspruch 1, dadurch gekennzeichnet, daß das stirnseitige Element auf der Außenseite der äußeren Folie und das Verstärkungsband auf der Innenseite der äußeren Folie befestigt ist.

## Claims

1. Hygiene article (9) of the nappy pants type, comprising an impermeable outer sheet (4), a permeable inner sheet, an absorbent pad arranged between the said two sheets, and two fastening systems making it possible to reclose the article around the user's waist, each system comprising a fastening tab secured laterally along one edge of the rear part of the outer sheet and at least one frontal element on the front part of the outer sheet, the said frontal element being capable of co-operating with the said fastening tab, involving a mechanical fastening system, the fastening tab (1) is provided with female elements (5), in particular of the loop type, and the frontal element is provided with male elements (7), characterized in that the frontal element has a small width (e) which is markedly smaller than the width L₂ of the band carrying female elements which forms the fastening tab (1); and in that the fastening system comprises at least one reinforcing band (8) which is secured to the front part (6) of the outer sheet (4) in line with the frontal element or elements and over a width greater than this or these.

2. Hygiene article according to Claim 1, characterized in that the male fastening means (7) are attached to the reinforcing band (8), the latter being secured to the external face of the outer sheet (4).

3. Hygiene article according to Claim 1, characterized in that the frontal element is secured to the external face of the outer sheet and the reinforcing band is secured to the internal face of the outer sheet.
